# EUROPÄISCHE PATENTSCHRIFT

(11) **EP 1 520 869 B1**
(45) Veröffentlichungstag und Bekanntmachung des Hinweises auf die Patenterteilung: **27.01.2010**
(21) Anmeldenummer: 04021317.5
(22) Anmeldetag: 08.09.2004
(51) Int. Cl.: C08G 64/30, C08G 64/02

(54) **Verfahren zur Herstellung von aliphatischen Oligocarbonatpolyolen**
Process for the preparation of aliphatic oligocarbonate polyols
Procédé de préparation de polyols d'oligocarbonate aliphatiques

(30) Priorität: 19.09.2003 DE 10343472
(43) Veröffentlichungstag der Anmeldung: 06.04.2005
(73) Patentinhaber: Bayer MaterialScience AG, 51368 Leverkusen (DE)
(72) Erfinder: Hofacker, Steffen, Dr., 51519 Odenthal (DE); Witossek, Herbert, Dr., 04157 Leipzig (DE); Bäcker, Lothar, 06773 Gossa (DE)

(56) Entgegenhaltungen:
- DE-A1- 10 027 907
- DE-A1- 10 130 882

## Beschreibung

Die vorliegende Erfindung betrifft ein verbessertes Verfahren zur Herstellung von aliphatischen Oligocarbonatpolyolen durch Umesterung von Dimethylcarbonat (DMC) mit einer aliphatischen Polyolkomponente.

Oligocarbonatpolyole können prinzipiell aus aliphatischen Polyolen durch Umsetzung mit Phosgen, bis-Chlorkohlensäureestern, Diarylcarbonaten, cyclischen Carbonaten oder Dialkylcarbonaten hergestellt werden. Sie sind wichtige Vorprodukte zur Herstellung von Kunststoffen, Lacken und Klebstoffen. Sie werden z.B. mit Isocyanaten, Epoxiden, (cyclischen) Estern, Säuren oder Säureanhydriden umgesetzt.

DE-A 101 30 882 beschreibt einen zweistufigen Prozess zur Herstellung von Oligocarbonatdilen, bei dem zunächst bei einem Druck von 1,5 bis 100 bar und einer Temperatur von 100°C bis 300°C Dimethylcarbonat (DMC) mit einem oder mehreren aliphatischen Diolen umgesetzt wird, wobei das bei der Reaktion gebildete Methanol zusammen mit DMC als Gemisch aus der Reaktion entfernt wird. Im zweiten Schritt erfolgt die Entkappung der endständigen Hydroxylgruppen durch Anlegen von Drücken von 1 bis 1000 mbar und Temperaturen von 160°C bis 250°C für mehrere Stunden. Die bevorzugte Reaktionstemperatur für den Entkappungsschritt liegt bei 200°C und der Druck bei 100 bis 200 mbar. Je nach Variante beträgt die Verweilzeit der Reaktionsmischung bei 200°C zwischen 9 und 50 Stunden. Die so hergestellten Oligocarbonatdiole weisen bei einem zahlenmittleren Molekulargewicht Mₙ von 2000 g/mol eine OH-Zahl (OHZ) von rund 56 mg KOH/g auf. Die tatsächliche OH-Funktionalität der so erhaltenen Produkte weicht jedoch vom theoretischen Wert von 2,00 ab. Grund hierfür ist die Bildung von Nebenprodukten mit unerwünschten Endgruppen, welche die Funktionalität erniedrigen, z.B. Methylester-, Methylether-, Vinyl-Gruppen und anderen.

Bei vielen Folgeanwendüngen, in denen Oligocarbonatpolyole eingesetzt werden ist neben der OHZ die tatsächliche OH-Funktionalität (f_{OH}) und vor allem deren Konstanz von besonderer Bedeutung. Weicht die Funktionalität um mehr als 0,10. Punkte vom theoretischen Wert (z.B. 2,00 für Oligocarbonatdiole) ab, so führt dies aufgrund der Anteile an monofunktionellen Oligocarbonaten, die in Polymerisationsreaktionen als Kettenabbrecher fungieren, zu Werkstoffen mit deutlich verschlechterten mechanischen Eigenschaften. Daher ist es notwendig, neben den klassischen Kenngrößen wie Viskosität, OHZ, Farbzahl etc. vor allem die tatsächliche OH-Funktionalität nahe dem Zielwert von z.B. 2,00 für bifunktionelle Oligocarbonatpolyole zu halten

Ferner weisen die in DE-A 101 30 882 beschriebenen Umesterungskatalysatoren bei der weiteren Umsetzung der Oligocarbonatdiole als Polyurethanrohstoff eine hohe Aktivität gegenüber Isocyanatgruppen-haltigen Verbindungen auf. Besonders ausgeprägt ist diese Eigenschaft bei Umsetzung von aromatischen (Poly)Isocyanaten mit Oligocarbonatpolyolen, welche mit Titanhaltigen Umesterungskatalysatoren hergestellt wurden, bei erhöhter Temperatur, wie dies beispielsweise bei der Herstellung von Gießelastomeren oder thermoplastischen Polyurethanen (TPU) der Fall ist. Dies kann dazu führen, dass die Topfzeit bzw. Reaktionszeit des Reaktionsgemisches derart verkürzt wird, dass eine Verwendung solcher Oligocarbonatpolyole für diese Anwendungsgebiete nicht mehr möglich ist. Um dies zu vermeiden, wird der im Produkt verbleibende Umesterungskatalysator nach abgeschlossener Synthese in mindestens einem zusätzlichen Produktionsschritt weitestgehend inaktiviert. In besonders sensiblen Anwendungsgebieten reicht aber selbst diese Inaktivierung nicht aus, um genügend lange Topfzeiten bzw. Reaktionszeiten zu erhalten.

Es wurde nun gefunden, dass sich durch eine mehrstufige Umesterung bei Temperaturen ≤ 170°C in Gegenwart von Ytterbium-Verbindungen als Umesterungskatalysatoren Oligocarbonatpolyole erhalten lassen, die diese Nachteile nicht mehr aufweisen.

Gegenstand der Erfindung ist ein Verfahren zur Herstellung von Oligocarbonatpolyolen, bei dem in einem ersten Schritt A) Dimethylcarbonat mit einer Polyolkomponente, die ein aliphatisches, Polyol oder ein Gemisch von aliphatischen Polyolen oder ein Gemisch von aliphatischen Polyolen mit Lactonen sein kann, unter Zusatz einer Ytterbiumverbindung als Umesterungskatalysator bei Drücken von Normaldruck bis 6 bar und Temperaturen von 100 bis 170°C, bevorzugt 100 bis 160°C, besonders bevorzugt 110 bis 155°C umgesetzt wird, wobei Schritt A) aus n ≥ 2 aufeinander folgenden Teilschritten Aᵢ), worin i Werte von 1 bis n annimmt, besteht, wobei in jedem Teilschritt Aᵢ) im Reaktor ein Druck ¹pᵢ von 3 bis 6 bar (absolut) eingestellt wird, der flüssigen Phase des Reaktionsgemisches ein Teil xᵢ der Gesamtmenge an Dimethylcarbonat zugegeben wird und nach einer Reaktionszeit tᵢ von 30 Minuten bis drei Stunden, bevorzugt 30 Minuten bis zwei Stunden, besonders bevorzugt 45 Minuten bis 90 Minuten bei einer Temperatur Tᵢ das bei der Reaktion gebildete Methanol in Form eines gasförmigen Methanol/Dimethylcarbonat-Gemisches durch Destillation aus dem Reaktionsgemisch entfernt wird, wobei bei konstanter Temperatur Tᵢ der Druck innerhalb von 2 bis 10 h, bevorzugt von 3 bis 8 h, besonders bevorzugt von 4 bis 6 h von ¹pᵢ auf einen Druck ²pᵢ von 1 bis 3 bar (absolut) reduziert wird, wobei im letzten Teilschritt Aₙ) die noch zur Gesamtmenge an Dimethylcarbonat fehlende Teilmenge xₙ zugegeben wird und der Druck ²pₙ am Ende des Teilschritts Normaldruck ist; und anschließend in einem zweiten Schritt B) der Druck weiter verringert und nach Erreichen eines Drucks ≤ 100 mbar in das Reaktionsgemisch ein Inertgasstrom eingeleitet wird, wobei parallel hierzu weiteres Methanol/Dimethylcarbonat-Gemisch abdestilliert wird, und wobei die Temperatur des Reaktionsgemisches einen Wert von 195°C, bevorzugt 190°C nicht übersteigt.

Der erste Schritt A) des erfindungsgemäßen Verfahrens besteht aus einer Abfolge von Teilschritten Aᵢ). Es werden n Teilschritte durchgeführt, wobei n einen Wert von mindestens 2 annimmt. In-einer bevorzugten Ausführungsform der Erfindung besteht der Verfahrensschritt A) aus drei Teilschritten A₁), A₂) und A₃). In jedem Teilschritt Aᵢ) wird der Reaktionsmischung ein Teil xᵢ der Gesamtmenge an DMC zugegeben, so dass nach dem letzten Teilschritt Aₙ), in dem die Teilmenge xₙ zugegeben wird, die Gesamtmenge an DMC der Reaktionsmischung zugesetzt wurde. Am Anfang jeden Teilschritts wird ein konstanter Druck ¹pᵢ im Bereich von 3 bis 6 bar (absolut) eingeregelt, bevorzugt durch Einleiten von Inertgas. Die Reaktionsmischung wird bei einer Temperatur Tᵢ im Bereich von 100°C bis 170°C für eine Reaktionszeit tᵢ getempert, dann wird das bei der Reaktion gebildete Methanol in Form eines gasförmigen Methanol/Dimethylcarbonat-Gemisches durch Destillation aus dem Reaktionsgemisch entfernt, wobei bei konstanter Temperatur Tᵢ der Druck von ¹pᵢ innerhalb von 4 bis 6 Stunden auf einen Druck ²pᵢ im Bereich von 1 bis 3 bar (absolut) reduziert wird. Meist wird die Destillation nach Erreichen des Drucks ²pᵢ noch etwa 30 bis 120 Minuten fortgesetzt. Anschließend wird der nächste Teilschritt Aᵢ₊₁) eingeleitet, wofür die Destillation bevorzugt unterbrochen wird. Jeder Teilschritt kann bei einer anderen Temperatur Tᵢ und anderen Drücken ¹pᵢ und ²pᵢ durchgeführt werden und es können unterschiedliche Reaktionszeiten tᵢ gewählt werden. Häufig werden jedoch für mehrere Teilschritte die gleichen Reaktionsbedingungen gewählt. Am Ende des letzten Teilschritts Aₙ) wird im Reaktor Normaldruck eingestellt, d.h. ²pₙ beträgt 1013 mbar.

Der zusätzliche Bedarf an DMC aufgrund des destillativen Verlustes während der Umesterungsphase beträgt 5 bis 50 %, bezogen auf die stöchiometrisch notwendige Menge. Die stufenweise Zugabe von DMC, der Einsatz von Ytterbiumverbindungen als Umesterungskatalysatoren und die somit mögliche niedrige Umsetzungstemperatur von maximal 170°C gewährleisten neben einer hohen Raum-Zeit-Ausbeute, dass die Nebenproduktbildung von Methyletherendgruppen stark vermindert bzw. vermieden werden kann, was zu einer Erhöhung der OH-Funktionalität beiträgt.

Dabei ist es erfindungswesentlich, dass die DMC-Konzentration im Reaktionsgemisch so eingestellt wird, dass einerseits die Nebenproduktbildung und der erforderliche DMC-Überschuss minimiert werden und andererseits die Raum-Zeit-Ausbeute nicht beeinträchtigt wird. Dies geschieht bevorzugt durch Konstanthalten des Drucks und der Temperatur, wodurch (temperaturabhängig) ein bestimmter Partialdruck des Dimethylcarbonates eingestellt wird. Der Gesamtdruck wird bevorzugt durch Einleiten von Inertgas in den Reaktor aufgebaut. Wichtig ist, dass die Zugabe des DMC derart erfolgt, dass der sich einstellende Eigendruck des DMC nicht den vorgegebenen Gesamtdruck übersteigt.

Es ist also darauf zu achten, dass die Teilmengen xᵢ, die Temperaturen Tᵢ und Reaktordrücke ¹pᵢ entsprechend aufeinander abgestimmt werden. Dadurch wird gewährleistet, dass sowohl die Nebenproduktbildung stark vermindert wird als auch der erforderliche stöchiometrische Überschuß an DMC minimiert werden kann.

Im zweiten erfindungsgemäßen Verfahrensschritt (Entkappung) wird der Druck weiter verringert und die Temperatur des Reaktionsgemisches auf Werte ≤ 195°C, bevorzugt ≤ 190°C eingestellt. Bei Erreichen eines Drucks ≤ 100 mbar wird in das Reaktionsgemisch ein Inertgasstrom eingeleitet. Dieser bewirkt neben den Vakuumbedingungen ein zusätzliches Herausschleppen von sich bildendem Methanol bzw. noch vorhandenem DMC. Durch dieses Vorgehen wird die Entstehung von zusätzlichen endständigen Hydroxylgruppen begünstigt, was zu einer Erhöhung der OH-Funktionalität und damit zu einer verbesserten Produktqualität beiträgt.

Als Inertgase kommen solche zum Einsatz, die in den chemischen Prozess nicht eingreifen. Beispiele sind Stickstoff, Argon, Methan und Erdgas. Bevorzugt wird Stickstoff eingesetzt.

Ein wesentliches Merkmal des Entkappungsschrittes liegt in der Temperaturkontrolle dieser Phase. Wird die Reaktionstemperatur auf mehr als 195°C gesteigert, so kommt es vermehrt zu Nebenproduktbildung. Im wesentlichen entstehen hierbei endständige Vinylgruppen, die zu einer drastischen Erniedrigung der OH-Funktionalität führen. Daher ist es für das erfindungsgemäße Verfahren wesentlich, die Temperatur während der Entkappungsphase auf Werte ≤ 195°C, bevorzugt ≤ 190°C einzustellen. So lässt sich die Bildung von endständigen Vinylgruppen vermeiden und damit die OH-Funktionalität erhöhen.

Nach einer Haltezeit zwischen 10 und 50 Stunden bei einem Druck ≤ 100 mbar und einer Temperatur ≤ 195°C, bevorzugt ≤ 190°C, wird der Druck durch Belüftung mit Inertgas, bevorzugt Stickstoff, auf Normaldruck erhöht. Anschließend können Produkteigenschaften wie OH-Zahl, Viskosität, zahlenmittleres Molekulargewicht, OH-Funktionalität usw. bestimmt werden.

Ist das zahlenmittlere Molekulargewicht des Produktes nach der Entkappungsphase zu hoch, so wird es durch Zugabe entsprechender Mengen Polyol und/oder Polyolmischung und erneutes Aufheizen des Reaktionsgemischs auf eine Temperatur von 100 bis 170°C (analog dem ersten Verfahrensschritt) für ein bis fünf Stunden korrigiert. Ein erneuter Entkappungsschritt ist nach dieser Korrektur nicht zwingend notwendig, sofern vor der Korrektur bereits alle endständigen OH-Gruppen frei vorlagen und durch Zugabe der Polyol-Komponente keine erneute Verkappung der endständigen Hydroxylgruppen aufgebaut wird.

Ist das zahlenmittlere Molekulargewicht des Produktes nach der Entkappungsphase zu niedrig, so wird es durch Zugabe entsprechender Mengen von DMC und erneutes Aufheizen des Reaktionsgemischs auf eine Temperatur von 100 bis 170°C (analog dem ersten Verfahrensschritt) für ein bis fünf Stunden korrigiert. Da dieses Vorgehen zu einem erneuten Aufbau der Verkappung führt, schließt sich ein erneuter Entkappungsschritt (analog dem zweiten Verfahrensschritt) an. Die Laufzeiten der erneuten Entkappung können jedoch aufgrund der verhältnismäßig kleinen zugesetzten DMC-Korrekturmenge stark reduziert werden. Wesentlich ist jedoch auch hier die Kontrolle der Temperatur analog dem oben beschrieben zweiten Verfahrensschritt.

Als Umesterungskatalysatoren werden im erfindungsgemäßen Verfahren Ytterbium-Verbindungen oder Mischungen davon eingesetzt. Beispiele sind Ytterbium(III)heptadionat, Ytterbium(III)acetylacetonat und Ytterbium(III)chlorid. Bevorzugt ist Ytterbium(III)acetylacetonat.

Die eingesetzten Katalysatorgehalte betragen 0,01 bis 1000 ppm, bevorzugt 0,1 bis 500 ppm, besonders bevorzugt 1 bis 200 ppm, bezogen auf die Gesamtmenge des hergestellten Oligocarbonatpolyols. Auf eine Deaktivierung des Katalysators kann verzichtet werden. Eine anschließende Maskierung, Fällung oder sonstige Entfernung oder Deaktivierung ist nicht notwendig.

Im erfindungsgemäßen Verfahren wird eine Polyolkomponente eingesetzt, die ein aliphatisches Polyol oder ein Gemisch von aliphatischen Polyolen oder ein Gemisch von aliphatischen Polyolen mit Lactonen ist.

Es können aliphatische Polyole mit 4 bis 50 C-Atomen in der Kette (verzweigt und unverzweigt), die auch durch zusätzliche Heteroatome wie Sauerstoff (O), Schwefel (S) oder Stickstoff (N) unterbrochen sein können, eingesetzt werden. Beispiele geeigneter Diole sind 1,4-Butandiol, 1,5-Pentandiol, 1,6-Hexandiol, 1,7-Heptandiol, 1,12-Dodecandiol, Cyclohexandimethanol, 3-Methyl-1,5-Pentandiol, 2,4-Diethyl-1,5-Pentandiol, Trimethylolpropan, Pentaerythrit, Bis-(2-hydroxyethyl)-ether, Bis-(6-hydroxyhexyl)-ether, durch Reduktion von dimerisierten ungesättigten Fettsäuren hergestellte Diole ("Dimerdiole"), kurzkettige C₂- oder C₃-Polyetherdiole mit einem zahlenmittleren Molekulargewicht < 500 g/mol oder kurzkettige C₄-Polyetherdiole mit einem zahlenmittleren Molekulargewicht < 700 g/mol sowie Mischungen daraus.

Weiterhin können die Additionsprodukte der Polyole mit Lactonen (Esterpolyole), z.B. ε-Caprolacton, Valerolacton etc., sowie Mischungen der Polyole mit Lactonen eingesetzt werden wobei eine einleitende Umesterung von Lacton und Polyol nicht nötig ist.

Bevorzugt werden in dem erfindungsgemäßen Verfahren 1,6-Hexandiol und/oder 1,5-Pentandiol und/oder 1,4-Butandiol, 1,12-Dodecandiol und/oder Cyclohexandimethanol eingesetzt. Besonders bevorzugt werden 1,6-Hexandiol, 1,5-Pentandiol oder 1,4-Butandiol eingesetzt; ganz besonders bevorzugt 1,6-Hexandiol.

Das erfindungsgemäße Verfahren ermöglicht die Herstellung von Oligocarbonatpolyolen, Oligocarbonatesterpolyolen, Oligocarbonatetherpolyolen oder Mischungen daraus mit zahlenmittleren Molekulargewichten von 500 bis 5000 g/mol, bevorzugt 500 bis 2000 g/mol und einem Restgehalt an Methanol und DMC von jeweils < 0,10 mol-%, bevorzugt < 0,05 mol-% und Gehalten an endständigen Vinylgruppen < 0,10 mol-%, bevorzugt < 0,05 mol-% und endständigen Methylethergruppen < 3,0 mol-%, bevorzugt < 2,5 mol-%. Die angegebenen mol.-%-Gehalte können als Anteile der aufgeführten Verbindung bezogen auf 1 mol der theoretischen Zielverbindung mit endständigen Hydroxylgruppen angesehen werden.

Werden Oligocarbonatdiole hergestellt, so beträgt deren OH-Funktionalität 1,85 bis 2,00, bevorzugt 1,90 bis 2,00, besonders bevorzugt 1,95 bis 2,00.

Die nach dem erfindungsgemäßen Verfahren hergestellten Oligocarbonatpolyole können mit Isocyanaten, Epoxiden, (cyclischen) Estern, Säuren oder Säurenhydriden umgesetzt und damit beispielsweise zur Herstellung von Polymeren, Kunststoffen, Fasern, Beschichtungen, Lacken und Klebstoffen eingesetzt werden. Ebenso können die erfindungsgemäß hergestellten Oligocarbonatpolyole als Bestandteile in polyurethanbasierten Gießelastomeranwendungen verwendet werden. Darüber hinaus eignen sie sich als Bausteine für feuchtigkeitshärtende Beschichtungen, als Bindemittel oder Bindemittelbestandteile und/oder Reaktivverdünner in lösemittelhaltigen oder wässrigen Polyurethanbeschichtungen. Sie können weiterhin als Bausteine für freie NCO-Gruppen enthaltende Polyurethanprepolymere oder in Polyurethandispersionen oder Polyurethanlösungen eingesetzt werden. Die nach dem erfindungsgemäßen Verfahren hergestellten Oligocarbonatpolyole können auch zur Herstellung thermoplastischer Kunststoffe wie aliphatischer und/oder aromatischer Polycarbonate, thermoplastischer Polyurethane etc. eingesetzt werden.

### Beispiele

Die in den Beispielen aufgeführten Gehalte an Verbindungen, die abweichend von der theoretischen. Zielverbindung nur eine oder keine endständigen Hydroxylgruppen tragen, wurden ebenso wie das zahlenmittlere Molekulargewicht über ¹H-NMR-Analytik und die integrale Auswertung der entsprechenden Signale ermittelt. Die in mol-% angegebenen Gehalte können als Anteile der aufgeführten Verbindung bezogen auf 1 mol der theoretischen Zielverbindung mit zwei endständigen Hydroxylgruppen angesehen werden. Bei der Berechnung der OH-Funktionalität werden die so ermittelten Werte als Anteile von Kettenabrechermolekülen aufgefasst und entsprechend berücksichtigt.

### Beispiel 1 (Partie 07700420, VPLS 2391)

14940 kg 1,6-Hexandiol, 5292 kg Dimethylcarbonat und 3,6 kg ytterbium(III)acetylacetonat wurden bei 70°C im gerührten Reaktor mit Kolonne und Totalkondensator vorgelegt. Anschließend wurde der Kesseldruck durch Einleiten von Stickstoff auf 5,2 bar absolut erhöht und konstant geregelt und der Kesselinhalt wurde auf 150°C aufgeheizt. Die Reaktionsmischung wurde 1 h bei 150°C unter Rückfluss gehalten. Anschließend wurde der Druck bei konstanter Temperatur in 5 h auf 2,2 bar abgesenkt und das anfallende, aus Methanol mit Anteilen von Dimethylcarbonat bestehende Destillat in eine Vorlage ausgeschleust. Danach wurde noch 1 h lang Methanol/Dimethylcarbonat-Gemisch bei 2,2 bar abdestilliert. Anschließend wurde der Druck erneut auf 5,2 bar erhöht, und weitere 5292 kg Dimethylcarbonat wurden zugegeben. Nach 1 h unter Rückfluss wurde wiederum unter Druckabsenkung auf 2,2 bar innerhalb von 5 h anfallendes Destillat bei konstanter Temperatur ausgeschleust. Nach 1 h bei 2,2 bar und 150°C wurde die Temperatur auf 140°C abgesenkt. Der Druck wurde mit Stickstoff auf 4,2 bar erhöht und 3699 kg Dimethylcarbonat wurden zugegeben. Nach 1 h unter Rückfluss wurde schließlich unter Druckabsenkung auf Normaldruck (1,01 bar) innerhalb von 5 h bei konstanter Temperatur anfallendes Destillat ausgeschleust.

Der Reaktordruck wurde dann zur Vervollständigung der Reaktion auf 90 mbar absolut abgesenkt. Nach Erreichen von 90 mbar wurden 4 m³/h Stickstoff über ein Tauchrohr in die Reaktionsmischung eingeleitet, um restliches Methanol zu entfernen; weiterhin wurde das Vakuum bis ca. 30 mbar abgesenkt. Nach 4 h wurde die Reaktortemperatur auf 170°C, nach weiteren 4 h auf 190°C erhöht. OHZ und Schmelzviskosität bei 75°C wurden alle 4 h bestimmt. Mit Nachsätzen von 280 kg, 200 kg und 65 kg 1,6-Hexandiol wurde der Ansatz korrigiert. Nach einer Gesamt-Entkappungszeit von 40 h bei einem Druck < 60 mbar und 190°C wurde der Ansatz auf 90°C abgekühlt und mit Stickstoff auf Normaldruck belüftet.

Man erhielt ein Oligocarbonatdiol mit folgenden Kennzahlen:

| | |
|---|---|
| zahlenmittleres Molgewicht: | 2000 g/mol |
| OH-Zahl: | 54,3 mg KOH/g |
| Viskosität bei 75°C: | 2620 mPas |
| Farbzahl (APHA): | 37 Hazen |
| OH-Funktionalität: | 1,96 |
| Gehalt an endständigen Vinylgruppen: | 0,0 mol-% |
| Gehalt an endständigen Methylethergruppen: | 1,3 mol-% |

### Vergleichsbeispiel 1

### (Partie 52)

415,1 kg 1,6-Hexandiol, 146,9 kg DMC sowie 100 g Ytterbium(III)-acetylacetonat wurden bei 70°C im gerührten Reaktor mit Kolonne und Totalkondensator vorgelegt. Nach zweimaligem Inertisieren durch Anlegen von Vakuum und Belüften mit Stickstoff wurde der Druck mit Stickstoff auf 5,2 bar (absolut) erhöht und mittels Druckregelung konstant geregelt. Anschließend wurde der Ansatz auf' 150°C aufgeheizt. Nach Erreichen der Temperatur wurde die Reaktionsmischung 2 h unter Rückfluss gehalten. Danach wurde 2 h lang ein aus Methanol und Dimethylcarbonat bestehendes Gemisch in eine Vorlage abdestilliert. Anschließend wurde der Druck innerhalb von 4 h bei konstanter Temperatur von 150°C auf 3 bar abgesenkt und dabei weiteres Destillat entfernt. Schließlich wurde der Druck wieder auf 5,2 bar erhöht und es wurden weitere 146,9 kg Dimethylcarbonat zugegeben. Bei konstanter Temperatur von 150°C wurde der Reaktionsansatz 2 h unter Rückfluss gehalten. Danach wurde wiederum 2 h lang ein aus Methanol und Dimethylcarbonat bestehendes Gemisch über Kopf in eine Vorlage abdestilliert. Anschließend wurde der Druck bei konstanter Temperatur von 150°C innerhalb von 4 h auf 3 bar und innerhalb von weiteren 8 h auf 2,5 bar abgesenkt; dabei wurde weiteres Destillat entfernt. In einem weiteren Umesterungszyklus wurde der Druck erneut auf 5,2 bar erhöht, und weitere 117,5 kg Dimethylcarbonat wurden zugegeben. Der Ansatz wurde 2 h unter Rückfluss gehalten. Danach wurde wiederum 2 h lang ein aus Methanol und Dimethylcarbonat bestehendes Gemisch über Kopf in eine Vorlage abdestilliert. Anschließend wurde der Druck bei konstanter Temperatur von 150°C in 7,5 h auf Normaldruck abgesenkt und dabei weiteres Destillat entfernt.

Die Temperatur wurde auf 180°C erhöht und der Druck auf ≤ 80 mbar gesenkt. Gleichzeitig wurde Stickstoff (125 1/h) über ein Durchleitrohr in die Reaktionsmischung eingeleitet. Nach 18 h Rühren bei 180°C wurde die Temperatur auf 200°C erhöht und für 40 h gehalten. Während dieser Phase wurde der Ansatz mit einem Nachsatz von 8,4 kg 1,6-Hexandiol korrigiert. Nach einer Gesamt-Entkappungszeit von 40 h bei 200°C und einem Druck < 60 mbar wurde der Ansatz auf 90°C abgekühlt und mit Stickstoff auf Normaldruck belüftet.

Man erhielt ein Oligocarbonatdiol mit folgenden Kennzahlen:

| | |
|---|---|
| zahlenmittleres Molgewicht: | 1800 g/mol |
| OH-Zahl: | 58,7 mg KOH/g |
| Viskosität bei 75°C: | 2200 mPas |
| Farbzahl (APHA): | 138 Hazen |
| OH-Funktionalität: | 1,88 |
| Gehalt an endständigen Vinylgruppen: | 3,3 mol-% |
| Gehalt an endständigen Methylethergruppen: | 1,5 mol-% |

### Vergleichsbeispiel 2

### (Partie 26)

8,7 kg 1,6-Hexandiol sowie 0,12 g Ytterbium(III)-heptadionat wurden im gerührten Reaktor mit Kolonne und Totalkondensator vorgelegt. Nach zweimaligem Inertisieren durch Anlegen von Vakuum und Belüften mit Stickstoff wurde der Druck mit Stickstoff auf 5,2 bar (absolut) erhöht und der Ansatz auf 185°C aufgeheizt. Mit Hilfe einer Druckregelung wurden 5,2 bar konstant geregelt. Nach Erreichen der Temperatur wurden über ein Tauchrohr in 11h 8,5 kg Dimethylcarbonat zudosiert. Gleichzeitig wurde ein aus Methanol und Dimethylcarbonat bestehendes Destillat über Kopf in eine Vorlage abdestilliert. Nach Ende der Zugabe wurde 2 h bei 185°C nachgerührt.

Bei einer Temperatur von 185°C wurde der Druck zunächst auf Normaldruck und dann weiter auf ca. 60 mbar abgesenkt, wobei weiterhin Destillat über Kopf abdestilliert wurde. Über ein Durchleitrohr wurde dann Stickstoff (10 1/h) in die Reaktionsmischung eingeleitet. Der Ansatz wurde 10 h unter diesen Bedingungen gerührt. Anschließend wurde mit Stickstoff belüftet und der Ansatz mit einem Nachsatz von 0,25 kg 1,6-Hexandiol korrigiert, danach wurde bei 180°C und einem Druck < 60 mbar weitere 10 h gerührt. Danach wurde der Ansatz auf 100°C abgekühlt und mit Stickstoff auf Normaldruck belüftet.

Man erhielt ein Oligocarbonatdiol mit folgenden Kennzahlen: (Bitterfelder Daten)

| | |
|---|---|
| zahlenmittleres Molgewicht: | 1750 g/mol |
| OH-Zahl: | 57,2 mg KOH/g |
| Viskosität bei 75°C: | 2200 mPas |
| Farbzahl (APHA): | 126 Hazen |
| OH-Funktionalität: | 1,78 |
| Gehalt an endständigen Vinylgruppen: | 0,0 mol-% |
| Gehalt an endständigen Methylethergruppen: | 10,9 mol-% |

## Patentansprüche

1. Verfahren zur Herstellung von Oligocarbonatpolyolen, bei dem
in einem ersten Schritt A) Dimethylcarbonat mit einer Polyolkomponente unter Zusatz einer Ytterbiumverbindung als Umesterungskatalysator bei Drücken von Normaldruck bis 6 bar und Temperaturen von 100 bis 170°C umgesetzt wird,
wobei Schritt A) aus n ≥ 2 aufeinander folgenden Teilschritten Aᵢ), worin i Werte von 1 bis n annimmt, besteht,
wobei in jedem Teilschritt Aᵢ) im Reaktor ein Druck ¹pᵢ von 3 bis 6 bar eingestellt wird, der flüssigen Phase des Reaktionsgemisches ein Teil xᵢ der Gesamtmenge an Dimethylcarbonat zugegeben wird und nach einer Reaktionszeit, tᵢ bei einer Temperatur Tᵢ das bei der Reaktion gebildete Methanol in Form eines gasförmigen Methanol/Dimethylcarbonat-Gemisches durch Destillation aus dem Reaktionsgemisch entfernt wird, wobei bei konstanter Temperatur Tᵢ der Druck von ¹pᵢ auf einen Druck ²pᵢ reduziert wird, und
wobei im letzten Teilschritt Aₙ) die noch zur Gesamtmenge an Dimethylcarbonat fehlende Teilmenge xₙ zugegeben wird und der Druck ²pₙ am Ende des Teilschritts Normaldruck ist;
und anschließend in einem zweiten Schritt B) der Druck weiter verringert wird und nach Erreichen eines Drucks ≤ 100 mbar in das Reaktionsgemisch ein Inertgasstrom eingeleitet wird, wobei parallel hierzu weiteres Methanol/ Dimethylcarbonat-Gemisch abdestilliert wird, und wobei die Temperatur des Reaktionsgemisches einen Wert von 195°C nicht übersteigt.

2. Verfahren gemäß Anspruch 1, bei dem als Umesterungskatalysator Ytterbium(III)acetylacetonat eingesetzt wird.

3. Verfahren gemäß Anspruch 1 oder 2, bei dem die Polyolkomponente 1,6-Hexandiol ist.

## Claims

1. Process for the preparation of oligocarbonatepolyols, wherein
in a first step A), dimethyl carbonate is reacted with a polyol component at pressures between normal pressure and 6 bar and temperatures of 100 to 170°C, a ytterbium compound being added as a transesterification catalyst,
in which step A) consists of n ≥ 2 consecutive partial steps Aᵢ), where i has values of 1 to n,
in which, in each partial step Aᵢ), a pressure ¹pᵢ of 3 to 6 bar is established in the reactor, a fraction xᵢ of the total amount of dimethyl carbonate is added to the liquid phase of the reaction mixture and, after a reaction time tᵢ at a temperature Tᵢ, the methanol formed in the reaction is removed from the reaction mixture in the form of a gaseous methanol/dimethyl carbonate mixture by distillation, the pressure being reduced from ¹pᵢ to ²pᵢ at a constant temperature Tᵢ, and
in which, in the last partial step Aₙ), the remaining fraction xₙ of the total amount of dimethyl carbonate is added and the pressure ²pₙ at the end of the partial step is normal pressure;
and then, in a second step B), the pressure is further reduced and, after it has reached ≤ 100 mbar, a stream of inert gas is introduced into the reaction mixture, further methanol/dimethyl carbonate mixture being distilled off in parallel and the temperature of the reaction mixture not exceeding a value of 195°C.

2. Process according to Claim 1 wherein the transesterification catalyst used is ytterbium(III) acetylacetonate.

3. Process according to Claim 1 or 2 wherein the polyol component is 1,6-hexanediol.

## Revendications

1. Procédé pour la préparation d'oligocarbonatepolyols, dans lequel
on fait réagir dans une première étape A) du carbonate de diméthyle avec un constituant polyol en utilisant un composé d'ytterbium comme catalyseur de trans-estérification à des pressions allant de la pression atmosphérique jusqu'à 6 bars et à des températures de 100 à 170°C,
l'étape (A) étant constituée de n ≥ 2 étapes partielles consécutives Aᵢ), où i prend les valeurs de 1 à n,
dans chaque étape partielle Aᵢ) une pression ¹pᵢ de 3 à 6 bars étant ajustée dans le réacteur, la phase liquide du mélange réactionnel étant additionnée d'une partie xᵢ de la quantité totale de carbonate de diméthyle et le méthanol formé dans la réaction étant éliminé après une durée de réaction tᵢ à une température Tᵢ dans la forme d'un mélange gazeux de méthanol/carbonate de diméthyle par distillation du mélange réactionnel, la pression étant à température constante Tᵢ réduite de ¹pᵢ à une pression ²pᵢ, et
dans la dernière étape partielle Aₙ) on ajoute la quantité partielle xₙ de carbonate de diméthyle faisant encore défaut à la quantité totale et la pression ²pₙ étant à la fin de l'étape partielle la pression atmosphérique ;
et on réduit encore ensuite la pression dans une seconde étape B) et on introduit dans le mélange réactionnel un courant de gaz inerte jusqu'à atteindre une pression ≤ 100 mbar, un autre mélange de méthanol/carbonate de diméthyle étant ici parallèlement éliminé par distillation, et la température du mélange réactionnel ne dépassant pas une valeur de 195°C.

2. Procédé selon la revendication 1, dans lequel on utilise comme catalyseur de trans-estérification de l'acétylacétonate d'ytterbium (III).

3. Procédé selon la revendication 1 ou 2, dans lequel le constituant polyol est le 1,6-hexanediol.
